# EUROPEAN PATENT APPLICATION

(11) **EP 1 747 759 A1**
(43) Date of publication of application: **31.01.2007**
(21) Application number: 06253933.3
(22) Date of filing: 27.07.2006
(51) Int. Cl.: A61B 17/34, A61B 17/00, A61B 17/28

(54) **Electroactive polymer-based flexing access port**

(30) Priority: 28.07.2005 US 161263
(71) Applicant: ETHICON ENDO-SURGERY, INC., Cincinnati, Ohio 45242 (US)
(72) Inventor: Ortiz, Mark S., Milford, Ohio 45150 (US)
(74) Representative: Tunstall, Christopher Stephen

(57) **Abstract**

Methods and devices are disclosed for providing access through tissue to a surgical site, such as anatomical cavities ranging in size from the abdomen to small blood vessels, such as veins and arteries, epidural, pleural and subarachnoid spaces, heart ventricles, as well as spinal and synovial cavities. In one exemplary embodiment, an access port is provided having one or more electrically expandable and contractible actuators that are adapted to change an orientation of the access port.

## Description

### FIELD OF THE INVENTION

The present invention relates broadly to surgical devices, and in particular to methods and devices for providing a flexing access port.

### BACKGROUND OF THE INVENTION

Access ports are widely used in medical procedures to gain access to anatomical cavities ranging in size from the abdomen to small blood vessels, such as veins and arteries, epidural, pleural and subarachnoid spaces, heart ventricles, and spinal and synovial cavities. The use of access ports has become more common as they provide minimally invasive techniques for establishing a portal for a number of procedures, such as those involving the abdominal cavity. Most access ports typically include a housing having a penetrating member extending therefrom and having a blunt or sharp tip for penetrating an anatomical cavity wall.

A trocar is one type of access post that is commonly used to provide a minimally invasive pathway for accessing a surgical site. Trocars generally include a cutting assembly (or obturator) that is disposed within an outer cannula (also referred to as the trocar tube or sleeve). The sharp distal end of the cutting assembly, with the cannula disposed therearound, is urged through the skin until it enters the anatomical cavity being penetrated. The cutting assembly is then withdrawn from the cannula, which provides a passageway through which access to the anatomical cavity is provided for other surgical devices.

While effective, current access ports can be difficult to manipulate directionally or angularly. In particular, most access ports are rigid to provide a straight path to a surgical site. While access ports having a curved configuration are available, such devices can be difficult to implant, and they can require the use of special complementary instruments.

Accordingly, there is a need for improved methods and devices for providing access to a surgical site, and in particular for providing an flexing access port that can be used with a variety of instruments.

### BRIEF SUMMARY OF THE INVENTION

The present invention generally provides methods and devices for providing access through tissue to a surgical site. In one exemplary embodiment, an access port is provided for allowing a directional orientation of at least one passageway extending therethrough to be adjusted as desired during use. While this can be achieved using a variety of techniques, in one embodiment the device can include a flexible elongate member with proximal and distal ends and a lumen extending therebetween, and at least one actuator coupled to at least a portion of the flexible elongate member. The at least one actuator is adapted to be selectively actuated to change a directional orientation of at least a portion of the flexible elongate member. While the actuator(s) can have a variety of configurations and can be coupled to the flexible elongate member to achieve certain orientational changes, in an exemplary embodiment, the actuator(s) can be adapted to change dimensionally, e.g., to expand radially and contract axially or to expand axially and contract radially, to change a directional orientation of the flexible elongate member.

As indicated above, the actuator(s) can be arranged in a variety of configurations in order effect a desired change in the directional orientation of the flexible elongate member. In one embodiment, the actuator can be axially disposed along a length of the flexible elongate member to cause bending along the axis thereof. Multiple actuators can also be in a spaced relationship around a circumference of the flexible elongate member to allow bending in multiple directions. In another embodiment, multiple actuators can be spaced longitudinally from one another to allow bending along certain portions of the flexible elongate member. In yet another embodiment, the actuators can be positioned at an angle relative to a central longitudinal axis of the flexible elongate member to cause bending and twisting of the flexible elongate member.

The actuators can also be formed from a variety of materials. In one exemplary embodiment, the actuator is in the form of an electroactive polymer (EAP). For example, the actuator can be in the form of a fiber bundle having a flexible conductive outer shell with several electroactive polymer fibers and an ionic fluid disposed therein. Alternatively, the actuator can be in the form of a laminate having at least one flexible conductive layer, an electroactive polymer layer, and an ionic gel layer. Multiple laminate layers can be used to form a composite. The actuator can also preferably include a return electrode and a delivery electrode coupled thereto, with the delivery electrode being adapted to deliver energy to each actuator from an external energy source.

In another exemplary embodiment, the access port can be formed from a plurality of electrically expandable and contractible actuators coupled to one another and defining a passageway therethrough. The actuators are adapted to change dimensionally when energy is delivered thereto to change a directional orientation of the passageway

Also disclosed herein are methods for providing access through tissue to a surgical site. In one embodiment, the method can include positioning a flexible access port through tissue to form a working channel to a surgical site, and selectively actuating at least one actuating member coupled to the flexible access port to cause the flexible access port to assume a non-linear configuration. For example, in one embodiment, the actuator can cause the flexible access port to bend. In other exemplary embodiments, multiple actuators can be selectively activated to move the flexible elongate member in multiple directions. For example, the method can include electrically actuating a first actuating member to cause the first actuating member to expand, thereby causing the flexible access port to move from a linear configuration to a first non-linear configuration. Energy delivery to the first actuating member can be terminated to cause the flexible access port to return to the resting linear configuration. The method can also include electrically actuating a second actuating member to cause the second actuating member to expand, thereby causing the flexible access port to move from the linear configuration to a second non-linear configuration that is be different from the first non-linear configuration. Energy delivery to the second actuating member can be terminated to cause the flexible access port to return to the linear resting position.

### BRIEF DESCRIPTION OF THE DRAWINGS

The invention will be more fully understood from the following detailed description taken in conjunction with the accompanying drawings, in which:

FIG. 1A is a cross-sectional view of a prior art fiber bundle type EAP actuator;

FIG. 1 B is a radial cross-sectional view of the prior art actuator shown in FIG. 1A;

FIG. 2A is a cross-sectional view of a prior art laminate type EAP actuator having multiple EAP composite layers;

FIG. 2B is a perspective view of one of the composite layers of the prior art actuator shown in FIG. 2A;

FIG. 3 is an exploded perspective view of one exemplary embodiment of a trocar and a flexible access port;

FIG. 4 is a perspective view of the trocar and flexible access port shown in FIG. 3 in a fully assembled configuration;

FIG. 5 is a perspective view of another exemplary embodiment of a flexible access port;

FIG. 6 is a cross-sectional view taken across line B-B of the flexible access port shown in FIG. 5;

FIG. 7 is a perspective view of another exemplary embodiment of a flexible access port;

FIG. 8A is a schematic showing the flexible access port of FIG. 3 positioned within tissue; and

FIG. 8B is a schematic showing the flexible access port of FIG. 3 activated to effect a lateral bend.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Disclosed herein are various methods and devices for providing access through tissue to a surgical site, such as anatomical cavities ranging in size from the abdomen to small blood vessels, such as veins and arteries, epidural, pleural and subarachnoid spaces, heart ventricles, as well as spinal and synovial cavities. In one exemplary embodiment, the access port can include one or more actuators that are adapted to change an orientation of the access port. In use, a directional orientation of the access port can be selectively adjusted to allow the access portion to receive a variety of surgical tools. A person skilled in the art will appreciate that access port can have a variety of configurations, and it can be adapted for use in a variety of medical procedures, including endoscopic, laparoscopic, arthroscopic and minimally invasive procedures. Moreover, the term "access port" is intended to include any device having a passageway extending therethrough, and it is not limited to cannulas. For example, the device can be in the form of a trocar or peroral sleeve that can be selectively shaped using one or more actuators for directional control of the trocar or sleeve.

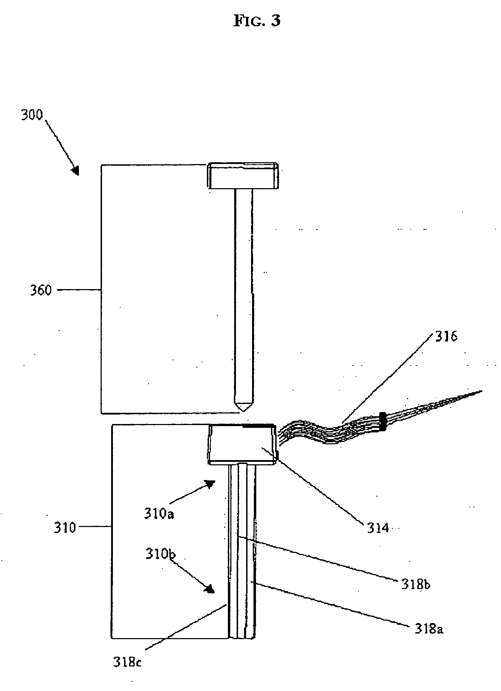

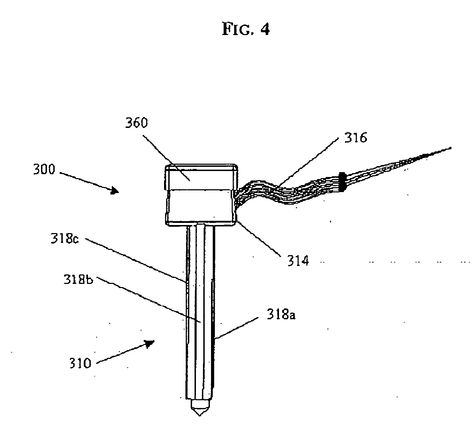

FIGS. 3-4 illustrate one exemplary embodiment of an access port 310 having multiple actuators 31 8a, 31 8b, 31 8c coupled thereto and adapted to alter a directional orientation of the access port 310. The access port 310 is shown in combination with a trocar 360 that can be placed within the access port 310 to facilitate insertion of the access port 310 into tissue. The use of a trocar is particularly advantageous as it can provide rigidity to a flexible access port. While the illustrated device 300 includes a trocar 360, a person skilled in the art will appreciate that the access port 310 can be used without the trocar 360 and/or that it can be incorporated into or used in connection with other surgical devices.

The access port 310 can have a variety of configurations, but in one exemplary embodiment the access port 310 can be in the form of a flexible elongate tube or cannula having proximal and distal ends 31 0a, 31 0b with an inner lumen extending therethrough and adapted to receive medical instruments therein. The shape and size of the access port 31 0 can vary depending upon the intended use. As shown in FIGS. 3-4, the access port 310 has a generally elongate cylindrical shape. The access port 310 can also include features to facilitate positioning thereof relative to a tissue surface. For example, as shown, the access port 310 has a flange 314 formed on the proximal end 31 0a thereof and adapted to limit insertion of the access port 310 into the tissue. The access port 310 can also include a variety of other features known in the art.

The materials used to form a flexible and/or elastic access port 310 can also vary. In certain exemplary embodiments, the access port 310 is preferably formed from a biocompatible polymer, such as silicone or latex. Other suitable biocompatible elastomers include, by way of non-limiting example, synthetic polyisoprene, chloroprene, fluoroelastomer, nitrile, and fluorosilicone. A person skilled in the art will appreciate that the materials can be selected to obtain the desired mechanical properties.

As previously indicated, the access port 310 can also include one or more actuators coupled to at least a portion thereof for changing an orientation of the access port 310. While the actuators can have a variety of configurations, one suitable actuator is an electroactive polymer actuator. Electroactive polymers (EAPs), also referred to as artificial muscles, are materials that exhibit piezoelectric, pyroelectric, or electrostrictive properties in response to electrical or mechanical fields. In particular, EAPs are a set of conductive doped polymers that change shape when an electrical voltage is applied. The conductive polymer can be paired with some form of ionic fluid or gel using electrodes. Upon application of a voltage potential to the electrodes, a flow of ions from the fluid/gel into or out of the conductive polymer can induce a shape change of the polymer. Typically, a voltage potential in the range of about 1V to 4kV can be applied depending on the particular polymer and ionic fluid or gel used. It is important to note that EAPs do not change volume when energized, rather they merely expand in one direction and contract in a transverse direction.

One of the main advantages of EAPs is the ability to electrically control and fine-tune their behavior and properties. EAPs can be deformed repetitively by applying external voltage across the EAP, and they can quickly recover their original configuration upon reversing the polarity of the applied voltage. Specific polymers can be selected to create different kinds of moving structures, including expanding, linear moving, and bending structures. The EAPs can also be paired to mechanical mechanisms, such as springs or flexible plates, to change the effect of the EAP on the mechanical mechanism when voltage is applied to the EAP. The amount of voltage delivered to the EAP can also correspond to the amount of movement or change in dimension that occurs, and thus energy delivery can be controlled to effect a desired amount of change.

There are two basic types of EAPs and multiple configurations for each type. The first type is a fiber bundle that can consist of numerous fibers bundled together to work in cooperation. The fibers typically have a size of about 30-50 microns. These fibers may be woven into the bundle much like textiles and they are often referred to as EAP yarn. In use, the mechanical configuration of the EAP determines the EAP actuator and its capabilities for motion. For example, the EAP may be formed into long strands and wrapped around a single central electrode. A flexible exterior outer sheath will form the other electrode for the actuator as well as contain the ionic fluid necessary for the function of the device. When voltage is applied thereto, the EAP will swell causing the strands to contract or shorten. An example of a commercially available fiber EAP material is manufactured by Santa Fe Science and Technology and sold as PANION™ fiber and described in U.S. Pat. No. 6,667,825, which is hereby incorporated by reference in its entirety.

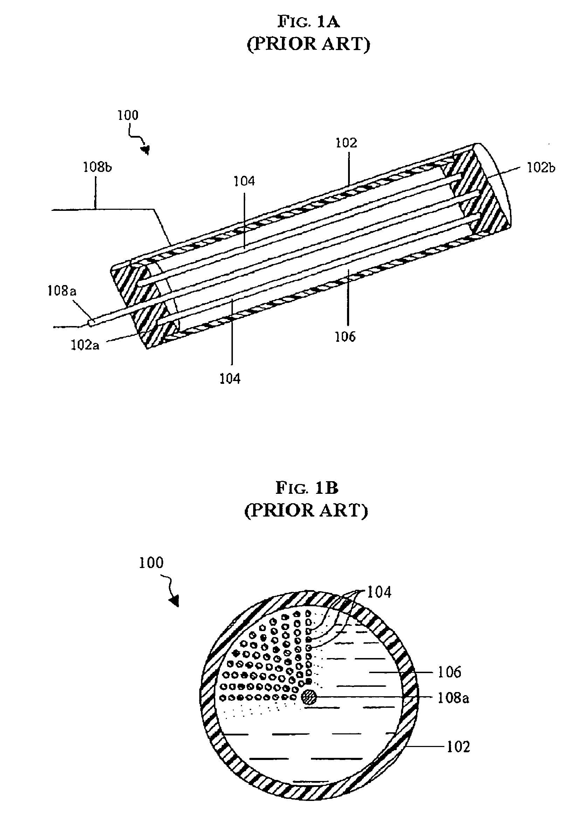

FIGS. 1A and 1B illustrate one exemplary embodiment of an EAP actuator 100 formed from a fiber bundle. As shown, the actuator 100 generally includes a flexible conductive outer sheath 102 that is in the form of an elongate cylindrical member having opposed insulative end caps 102a, 102b formed thereon. The conductive outer sheath 102 can, however, have a variety of other shapes and sizes depending on the intended use. As is further shown, the conductive outer sheath 102 is coupled to a return electrode 1 08a, and an energy delivering electrode 108b extends through one of the insulative end caps, e.g., end cap 102a, through the inner lumen of the conductive outer sheath 102, and into the opposed insulative end cap, e.g., end cap 102b. The energy delivering electrode 108b can be, for example, a platinum cathode wire. The conductive outer sheath 102 can also include an ionic fluid or gel 106 disposed therein for transferring energy from the energy delivering electrode 108b to the EAP fibers 1 04, which are disposed within the outer sheath 102. In particular, several EAP fibers 104 are arranged in parallel and extend between and into each end cap 102a, 120b. As noted above, the fibers 104 can be arranged in various orientations to provide a desired outcome, e.g., radial expansion or contraction, or bending movement. In use, energy can be delivered to the actuator 100 through the active energy delivery electrode 108b and the conductive outer sheath 102 (anode). The energy will cause the ions in the ionic fluid to enter into the EAP fibers 104, thereby causing the fibers 104 to expand in one direction, e.g., radially such that an outer diameter of each fiber 104 increases, and to contract in a transverse direction, e.g., axially such that a length of the fibers decreases. As a result, the end caps 102a, 1 20b will be pulled toward one another, thereby contracting and decreasing the length of the flexible outer sheath 102.

Another type of EAP is a laminate structure, which consists of one or more layers of an EAP, a layer of ionic gel or fluid disposed between each layer of EAP, and one or more flexible conductive plates attached to the structure, such as a positive plate electrode and a negative plate electrode. When a voltage is applied, the laminate structure expands in one direction and contracts in a transverse or perpendicular direction, thereby causing the flexible plate(s) coupled thereto to shorten or lengthen, or to bend or flex, depending on the configuration of the EAP relative to the flexible plate(s). An example of a commercially available laminate EAP material is manufactured by Artificial Muscle Inc, a division of SRI Laboratories. Plate EAP material, referred to as thin film EAP, is also available from EAMEX of Japan.

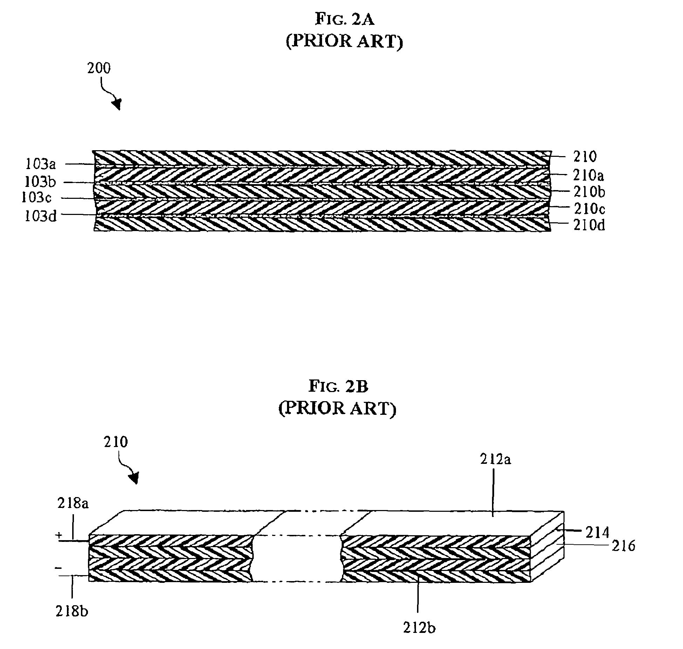

FIGS. 2A and 2B illustrate an exemplary configuration of an EAP actuator 200 formed from a laminate. Referring first to FIG. 2A, the actuator 200 can include multiple layers, e.g., five layers 210, 210a, 210b, 210c, 210d are shown, of a laminate EAP composite that are affixed to one another by adhesive layers 103a, 103b, 103c, 103d disposed therebetween. One of the layers, i.e., layer 210, is shown in more detail in FIG. 2B, and as shown the layer 210 includes a first flexible conductive plate 212a, an EAP layer 214, an ionic gel layer 216, and a second flexible conductive plate 212b, all of which are attached to one another to form a laminate composite. The composite can also include an energy delivering electrode 218a and a return electrode 218b coupled to the flexible conductive plates 212a, 212b, as further shown in FIG. 28. In use, energy can be delivered to the actuator 200 through the active energy delivering electrode 218a. The energy will cause the ions in the ionic gel layer 21 6 to enter into the EAP layer 214, thereby causing the layer 214 to expand in one direction and to contract in a transverse direction. As a result, the flexible plates 212a, 212b will be forced to flex or bend, or to otherwise change shape with the EAP layer 214.

Referring back to FIGS. 3-4, either type of actuator can be used with the access port 310 to allow an orientation of the access portion 310 to be directionally altered. In the illustrated embodiment, the access port 310 includes four actuators (only three actuators 31 8a, 31 8b, 318c are shown) coupled thereto. The actuators 31 8a, 31 8b, 318c are in the form of elongate bands that are coupled to an external surface of the access port 310. An adhesive or other mating techniques can be used to mate the actuators 318a, 318b, 31 8c to the access port 31 0. While not shown, the actuators 318a, 31 8b, 31 8c can optionally be disposed within the inner lumen of the access port 310, and/or they can be embedded within the walls of the access port 310. A person skilled in the art will appreciate that a variety of techniques can be used to couple the actuating members 31 8a, 31 8b, 31 8c to the access port 310.

The actuators 31 8a, 31 8b, 318c can also be coupled to the access port 310 in a variety of orientations to achieve a desired change in the orientation of the access port 310. In the illustrated embodiment, the actuators 318a, 31 8b, 31 8c extend along a substantial portion of a length of the access port 310, and they are spaced apart from one another around a diameter or circumference of the access port 310. Such a configuration allows the access port 310 to be directionally altered, e.g., bent, in one of four directions, each direction corresponding to a location of each actuating member 318a, 318b, 31 8c. In particular, in one embodiment the actuators 31 8a, 318b, 31 8c can be configured to expand axially and contract radially to increase a length of the access port 310, i.e., to push the opposed ends 310a, 310b of the access port 310 away from one another. Thus, when energy is delivered to one of the actuators 31 8a, 31 8b, 318c, the actuator 31 8a, 318b, 318c will cause the access port 310 to bend in a direction that is opposite to the location of the activated actuator. Alternatively, the actuators 31 8a, 31 8b, 318c can be configured to expand radially and contract axially to decrease a length of the access port 310, i.e., to draw the ends of the access port 310 coupled to the activated actuator toward one another. Thus, when energy is delivered to one of the actuators 31 8a, 31 8b, 318c, the actuator 318a, 31 8b, 318c will cause the access port 310 to bend in a direction in which the activated actuator is located.

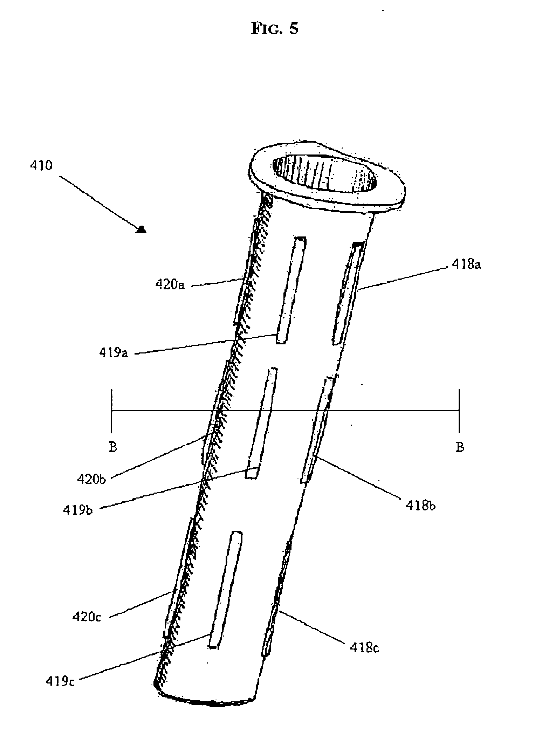

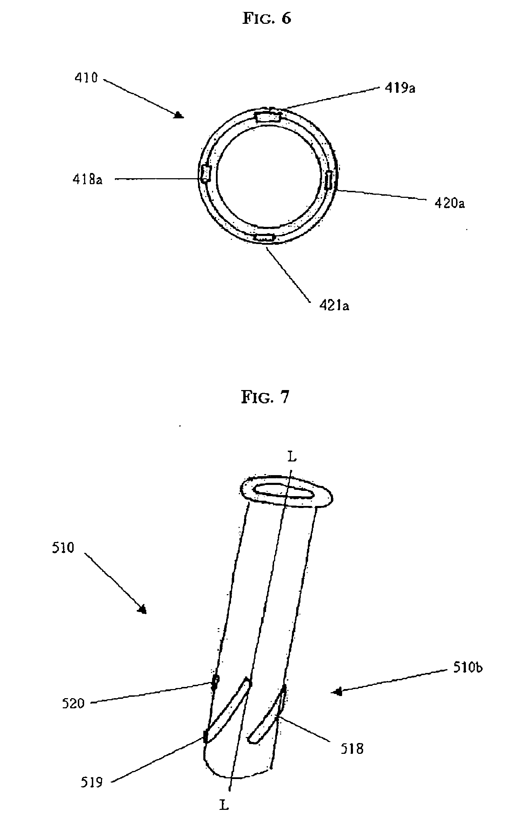

In another exemplary embodiment, the actuators can be arranged in a configuration to cause only a portion of the access port to change an orientation. FIG. 5 illustrates one such embodiment. As shown the access port 410 includes several EAP bands of actuators 41 8a, 41 8b, 41 8c, 41 9a, 41 9b, 41 9c, 420a, 420b, 420c arranged in columns along the length of the access port 410 and spaced apart from one another along the length, as well as around the circumference of the access port 410. FIG. 6 illustrates a cross-section of the access port 410 showing the actuators 41 8a, 41 9a, 420a, 421 a spaced relative to one another around the circumference of the access port 410.

The configuration shown in FIG. 5 allows a portion of the access port 410 to be selectively directionally oriented. in particular, as previously explained with respect to FIGS. 3-4, energy can be selectively delivered to one of the actuators 41 8a, 41 8b, 41 8c, 41 9a, 419b, 41 9c, 420a, 420b, 420c to cause the actuator to move the portion of the access port 410 to which the actuator is attached in a desired direction. For example, where the actuator is configured to expand axially and contract radially, the actuator will cause a portion of the access port 410 to bend in a direction opposite to the location of the activated actuator. Conversely, where the actuator is configured to expand radially and contract axially, the actuator will cause a portion of the access port 410 to bend in a direction in which the activated actuator is located. This configuration can be particularly effective where it is desirable to bend various portions, such as proximal, distal, and intermediate portions, of the actuators in different directions. A person skilled in the art will appreciate that the quantity and location of actuators can vary to achieve bending movement at desired locations along the actuator in desired directions.

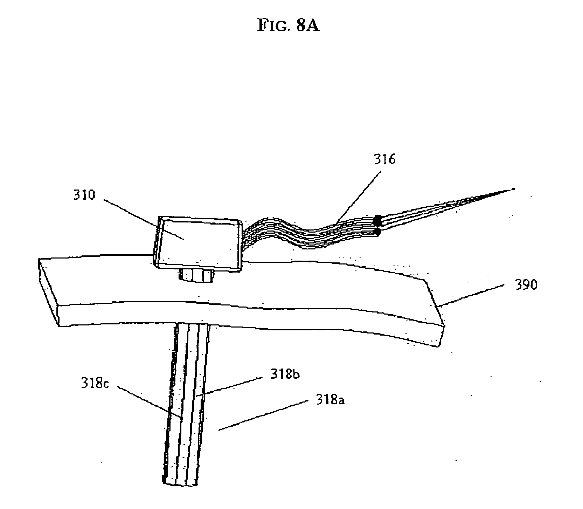

In another exemplary embodiment, the actuators can be arranged in a configuration such that a portion of the access port can bend and twist. For example, as shown in FIG. 7, elongate bands of actuators 518, 519, 520 can be disposed around the access port 510 at an angle relative to a longitudinal axis L of the access port 510. While the actuators 518, 519, 520 are shown positioned adjacent to the distal portion 510b of the access port 510, the actuators 518, 51 9, 520 can be located along any portion, or a substantial length of, the access port 510. In use, when energy is delivered to one of the actuators 51 8, 519, 520, the activated actuator can expand axially and contract radially, or conversely expand radially and contract axially, to cause the access port 510 to twist and bend in a direction opposite from a location of the actuator, or in a direction in which the actuator is located, respectively.

A person skilled in the art will appreciate that various combinations of the actuators shown in FIGS. 3-7 can be used, and that the actuators can have a variety of other configurations, shapes, and sizes, and that they can be positioned at virtually any orientation relative to the access port to provide desired directional changes to the access port. A person skilled in the art will also appreciate that the actuators can be configured to form an access port, rather than having a flexible access port coupled thereto.

In use, energy can be selectively delivered to one or more of the actuators through electrodes. In an exemplary embodiment, each actuator can be coupled to a return electrode and a delivery electrode that is adapted to communicate energy from an external power source to the actuator. The electrodes can be coupled to the actuators using a variety of techniques. For example, they can extend through the inner lumen in the tube and through a sidewall of the tube to mate to the actuator. Alternatively, they can be embedded in the sidewalls of the access tube, or they can extend along an external surface of the access tube. The electrodes can also be coupled to an external control mechanism that allows the user to select one or more actuators to deliver energy to. The external control mechanism can also be configured to allow a user to adjust the amount of energy being delivered, thereby allowing the degree of movement, e.g., bending, to be controlled. While the external control mechanism can be any mechanism known in the art, in one embodiment it can be in the form of a joystick such that movement of the joystick corresponds to a direction of bending movement of the access port.

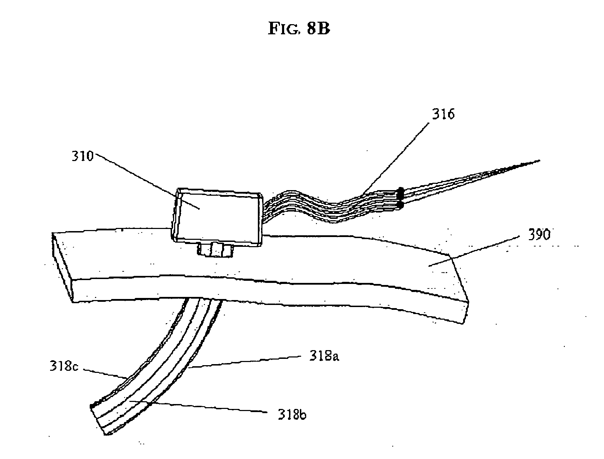

FIGS. 8A-8B illustrate one exemplary method of using an access port, e.g., access port 31 0 of FIG. 3, to provide access through tissue to a surgical site. As shown, the access port 310 is inserted through the skin 390 to provide access to a body cavity (not shown) through the lumen of the access port 310. Various techniques can be used to insert the access port 310 through the tissue, but in one exemplary embodiment, as previously described, a trocar 360 can be inserted through the access port 310 and the assembly can be penetrated through the skin 390 until it enters the body cavity. The trocar 360 is then withdrawn, leaving the access port 310 to provide a pathway through the tissue to the body cavity.

Once implanted, as shown in FIG. 8A, the access port 310 can be in a normal longitudinal or linear configuration with each of the actuators 318a, 31 8b, 31 8c being de-activated, i.e., in a resting configuration without energy being applied thereto. In order to facilitate the insertion of surgical tools through the access port 310, or to otherwise facilitate access to the surgical site, the access port 310 can be manipulated by laterally bending it in a desired direction, as shown in FIG. 8B. As explained above, this can be achieved by delivering electrical energy through one of the electrodes 316 to one of the actuators, e.g., actuator 318a, disposed on the access port 31 0 causing the actuator 31 8a to change dimensions and thereby bend the access port 310. The degree of bending can be controlled by adjusting the amount of energy being delivered, and the lateral bend can be maintained so long as the energy is continuously supplied to the actuator. Energy delivery to the actuator 318a can be terminated to cause the access port 310 to return to its resting configuration. Other actuators can also be selectively activated and de-activated, either alone or in combination, to change the directional orientation of the access port 310.

One skilled in the art will appreciate further features and advantages of the invention based on the above-described embodiments. For example, the access port can be provided in kits having access ports with different lengths to match a depth of the cavity of the working area of the patient. The kit may contain any number of sizes or alternatively, a facility, like a hospital, may inventory a given number of sizes and shapes of the access port. Accordingly, the invention is not to be limited by what has been particularly shown and described, except as indicated by the appended claims. All publications and references cited herein are expressly incorporated herein by reference in their entirety.

## Claims

1. A surgical access port, comprising:
a flexible elongate member having proximal and distal ends with a lumen extending therebetween; and
at least one actuator coupled to at least a portion of the flexible elongate member and adapted to change dimensionally when energy is delivered thereto to change a directional orientation of at least a portion of the flexible elongate member.

2. The access port of claim 1, wherein the at least one actuator is axially disposed along at least a portion of the flexible elongate member.

3. The access port of claim 1, further comprising a plurality of actuators positioned in a spaced relationship around a circumference of the flexible elongate member.

4. The access port of claim 3, wherein the plurality of actuators are spaced longitudinally from one another along a length of the flexible elongate member.

5. The access port of claim 1, wherein the at least one actuator is positioned at an angle relative to a central longitudinal axis of the flexible elongate member.

6. The access port of claim 1, wherein the at least one actuator is adapted to bend at least a portion of the flexible elongate member.

7. The access port of claim 1, further comprising a plurality of actuators, each actuator being adapted to bend the flexible elongate in a predetermined direction such that the flexible elongate member is adapted to bend in a plurality of predetermined directions.

8. The access port of claim 1, wherein the at least one actuator comprises an electroactive polymer.

9. The access port of claim 1, wherein the at least one actuator comprises a flexible conductive outer shell having an electroactlve polymer and an ionic fluid disposed therein.

10. The access port of claim 1 , wherein the at least one actuator comprises at least one electroactive polymer composite having at least one flexible conductive layer, an electroactive polymer layer, and an ionic gel layer.

11. A surgical access port, comprising:
a plurality of electrically expandable and contractible actuators coupled to one another and defining a passageway therethrough, the plurality of actuators being adapted to change dimensionally when energy is delivered thereto to change a directional orientation of the passageway.
